# EUROPEAN PATENT SPECIFICATION

(11) **EP 1 613 759 B1**
(45) Date of publication and mention of the grant of the patent: **01.08.2012**
(21) Application number: 04725049.3
(22) Date of filing: 01.04.2004
(51) Int. Cl.: C12P 1/06, C12P 1/04, C12P 19/62, C12R 1/01

(54) **Fermentation processes with low concentrations of carbon- and nitrogen-containing nutrients**
Fermentationsprozesse mit niedrigen Konzentrationen an Kohlenstoff- und Stickstoffhaltigen Nährstoffen
Procedes de fermentation presentant de faibles concentrations en nutriments contenant du carbone ou de l'azote

(30) Priority: 04.04.2003 EP 03100896
(43) Date of publication of application: 11.01.2006
(60) Divisional application: 10178179.7
(73) Proprietor: DSM IP Assets B.V., 6411 TE Heerlen (NL)
(72) Inventor: HOLLANDER, DE, Johannes, Abraham, NL-2343 NE Oegstgeest (NL); ESWILDER, Freddy, Ronald, NL-2645 HE Delfgauw (NL); ZANDEN, VAN DER, Paulus, Petrus, Maria, NL-2641 MS Pijnacker (NL)
(74) Representative: Pallard, Caroline Chantal Patricia
(86) International application number: PCT/EP2004/003662
(87) International publication number: WO 2004/087934

(56) References cited:
- EP-A- 0 199 499
- EP-A- 0 796 916
- US-A- 3 892 850
- US-A- 4 480 034
- US-A- 5 182 207
- US-A- 5 902 579
- JUNKER B ET AL: "Use of soybean oil and ammonium sulfate additions to optimize secondary metabolite production" BIOTECHNOL BIOENG;BIOTECHNOLOGY AND BIOENGINEERING DEC 5 1998 JOHN WILEY & SONS INC, NEW YORK, NY, USA, vol. 60, no. 5, 5 December 1998 (1998-12-05), pages 580-588, XP002287283
- FARID MOHAMED A ET AL: "Optimization of the cultivation medium for natamycin production by Streptomyces natalensis" JOURNAL OF BASIC MICROBIOLOGY, vol. 40, no. 3, 2000, pages 157-166, XP009033269 ISSN: 0233-111X
- CANEDO L M ET AL: "AB-400, a new tetraene macrolide isolated from Streptomyces costae" JOURNAL OF ANTIBIOTICS, JAPAN ANTIBIOTICS RESEARCH ASSOCIATION. TOKYO, JP, vol. 53, no. 6, June 2000 (2000-06), pages 623-626, XP009017204 ISSN: 0021-8820
- MADDEN T ET AL: "Organic acid excretion by Streptomyces lividans TK24 during growth on defined carbon and nitrogen sources" MICROBIOLOGY, SOCIETY FOR GENERAL MICROBIOLOGY, READING, GB, vol. 142, no. 11, November 1996 (1996-11), pages 3181-3185, XP001154955 ISSN: 1350-0872

## Description

### Field of the invention

The present invention relates to the field of fermentative production of natamycin.

### Background of the invention

The *actinomycetes,* a family of filamentous bacteria, are of great importance for the fermentation industry. Many members of this family are known to produce secondary metabolites or extracellular enzymes and several of these products of bacterial metabolism have an industrial application. For obtaining these products, the bacteria are generally cultivated in liquid media (submerged cultures), leading to excretion of the products into the liquid, from which they can be isolated. Formation of product can take place during the initial fast growth of the organism and/or during a second period in which the culture is maintained in a slow-growing or non-growing state. The amount of product which is formed per unit of time during such a process (the productivity) is generally a function of a number of factors: the intrinsic metabolic activity of the organism; the physiological conditions prevailing in the culture (e.g. pH, temperature and medium composition); and the amount of organisms which are present in the equipment used for the process. Generally, during optimisation of a fermentation process, it is preferred to obtain a concentration of bacteria that is as high as possible because, assuming that the intrinsic productivity per unit of organism is a constant, the highest titer of product will be obtained. However, one particular characteristic of the bacteria which belong to the family of *actinomycetes,* makes it difficult to achieve this goal. *Actinomycetes,* when grown in submerged culture, have a filamentous morphology, which generally leads to highly viscous culture fluids. A high viscosity of the culture limits the rate of oxygen transfer to the culture. Virtually all processes utilising *actinomycetes* depend on the presence and consumption of oxygen and therefore a limitation in oxygen transfer will impose a limitation on the overall process productivity. The viscosity of a culture fluid is determined by a number of factors such as the composition of the medium, the presence and nature of products excreted by the microorganisms, and

(most important) the morphology of the microorganism. If one could influence the morphological characteristics of the microorganisms in a positive way (i.e. to decrease the specific viscosity), the process could be operated at a higher production rate or a higher concentration of bacteria could be achieved. Both changes in the process would result in a higher productivity.

Junker et al. (1998), Biotechnology and Bioengineering, vol. 60, no. 5, p. 580-588 describes the use of soybean oil and ammonium sulphate additions to optimize the production of the immunoregulant L-683,590 by a valine―overproducing *Streptomyces hygroscopicus* mutant.

Farid et al. (2000), Journal of Basic Microbiology, vol. 40, no. 3, p. 157-166 describes the effects of certain nutrients on natamycin production by *Streptomyces natalensis* in submerged batch cultures.

EP 0 796 916 A describes a process for the fermentative production of an amino acid by using phosphorous limited or phosphorous/carbon double limited growth conditions.

EP 0 199 499 A describes a continuous process for the production of ethanol by fermentation with strains of Zymomonas.

US 5,182,207 describes new antibiotic compounds, called Antibiotics S541, which may be prepared by fermentation of *Streptomyces* organisms.

US 4,480,034 describes improved methods for the production and recovery of metabolic products in bioconversion systems.

Canedo et al. (2000), Journal of Antibiotics, vol. 53, no. 6, p. 623-626 describes a new tetraene macrolide, called AB-400, isolated from *Streptomyces costae.*

Madden et al. (1996), Microbiology, vol. 142, no. 11, p. 3181-3185 describes organic acid excretion by *Streptomyces lividans* TK24 during growth on defined carbon and nitrogen sources.

US 5,902,579 describes methods for preparing biomass containing 5-60% natamycin and its use in animal feed.

US 3,892,850 describes an antifungal antibiotic, called pimaricin, and its production by *Streptomyces natalensis.*

### Summary of the invention

The present invention provides a fermentation process for the production of natamycin comprising culturing a filamentous bacterial strain of the genus *Streptomyces* in a liquid fermentation medium, wherein the carbon containing nutrients and nitrogen containing nutrients are maintained at concentrations in the fermentation medium of less than 5 g/l and less than 0.5 g/l, respectively.

Preferably, a feed comprising carbon and nitrogen containing nutrients is supplied to the medium and the nutrients in the feed are such that the concentrations of both carbon and nitrogen containing nutrients are maintained in the culture at less than 5 g/l and 0.5 g/l, respectively.

### Detailed description of the invention

Surprisingly, it has been found that certain medium compositions lead to a reduced culture viscosity of a fermentation process comprising a filamentous bacterial strain of the genus *Streptomyces,* without affecting the production of natamycin. An important factor appears to be the ratio of nitrogen containing nutrients (N) to carbon containing nutrients (C) in the medium. A high N/C ratio (relative excess of nitrogen compounds) leads to viscous cultures, whereas a low N/C ratio results in relatively low viscosity of the culture fluid. When the amount of nitrogen in the medium is restricted too much, this leads to very poor growth of the organism and low amounts of product are formed. However, at an intermediate N/C ratio, growth of the organism is good and product formation is normal, while the morphology of the organism is apparently changed in such a way that the viscosity of the culture fluid is significantly reduced. The consequence of this finding is, that by carefully controlling the medium, or more specifically by controlling the ratio of carbon and nitrogen containing nutrients in the medium, a process comprising a filamentous bacterial strain of the genus *Streptomyces* can be improved significantly.

For example, the *Streptomyces* strains *Streptomyces natalensis* and *Streptomyces gilvosporeus* produce the antifungal compound natamycin, which has several applications as an antifungal compound. Fermentation processes comprising such filamentous bacteria are generally characterised by two phases. Usually the process starts with a phase where growth of the microorganism occurs until conditions for growth become unfavourable, for instance because one of the growth supporting nutrients becomes depleted from the medium. The initial (batch) phase may be followed by a phase where the microorganisms are maintained in a viable state. Often most of the product of interest is formed in this second phase. In this second phase, more nutrients may be supplied to the culture, either discontinuously as a single or repeated charge of fresh nutrients, or continuously by feeding one or more nutrients containing fluids into the fermentation vessel. This mode of fermentation is called fed-batch fermentation. Preferably, a fermentation process may be further prolonged by removing part of the fermentation mash, for instance when the fermentation vessel becomes completely filled as a result of feeding with nutrient containing fluids. This process form is called extended fermentation or repeated (fed-)batch fermentation.

The initial (batch) phase will end when one of the nutrients is depleted. This phase may be followed by measuring the oxygen uptake which will decrease towards the end of the initial phase. In general, the initial phase will take 6 to 48 hours. The second phase starts when feeding of the nutrients is started. Feeding of nutrients allows the continuation of the fermentation process for a longer period than is possible in a simple batch fermentation process.

In general, for each production process, the optimal ratio of carbon and nitrogen containing nutrients can be determined by the skilled person, depending on the elementary composition of the organism and the product(s), the effect of the N/C ratio on the physiology of the organism and, more specifically, the product forming capacity of the organism. It has been found that neither carbon excess nor nitrogen excess will lead to the desired result. In the optimal situation, both the available carbon and nitrogen will be almost depleted from the medium at the end of the batch process and/or during the process of prolonged fed-batch type fermentation. The concentration of the nitrogen containing nutrient in the medium during the second phase is less than 0.5 g/l, more preferably less than 0.25 g/l and most preferably less than 0.1 g/l (expressed as gram of nitrogen per litre). The concentration of the carbon containing nutrient is less than 5 g/l. more preferably less than 2.5 g/l and most preferably less than 1 g/l (expressed as gram of carbon per litre). The feed can be supplied as one feed containing all the nutrients or preferably as more than one subfeeds each comprising either a nitrogen containing nutrient, a carbon containing nutrient or a combination of nitrogen and carbon containing nutrients.

The feed is also controlled in such a way that the amount of oxygen is between 20 and 70% of air saturation, preferably between 30 and 60% of air saturation.

Oxygen, typically in the form of air, is generally introduced at or near the bottom of the fermentor. One of more nozzles are installed for the introduction of air or another oxygen containing gas such as (purified) oxygen.

Optionally, a stirrer is present in the reactor to stimulate the oxygen uptake. Moreover, the stirrer prevents concentration gradients of the feed or subfeed developing in the fermentor.

### Legend to the Figures

Figure 1: Viscosity development of a nitrogen excess-culture (•) and a nitrogen-carbon double-limited culture (◆).
Figure 2: Agitation power required to control the dissolved oxygen concentration at a 30% air saturation. Both cultures, nitrogen excess (•) and nitrogen-carbon double-limited (◆), were operated under otherwise similar process conditions.
Figure 3: Viscosity development of a nitrogen excess culture (•) and a nitrogen-carbon double-limited culture (◆).
Figure 4: Product accumulation in a nitrogen excess culture (•) and a nitrogen-carbon double-limited culture (◆).
Figure 5: Full scale fermentation of *Streptomyces natalensis* to produce natamycin. The initial process (•) used a limiting feed of soybean oil, while the NH3 concentration was kept at a non-limiting level. In the improved process (◆) the NH3 concentration was kept at a low value by continuous feeding of a NH3 solution in proportion to the oil feeding rate. The reduced culture viscosity allowed faster feeding of oil. The increase in product formation was approximately proportional to the increase in oil feeding rate.

### EXAMPLES

### Example 1

*Streptomyces natalensis* strain ATCC27448 was cultivated in 2000ml conical shake containing 500 mL growth medium of the following composition:

| | g/L |
|---|---|
| Glucose.1H₂O | 30 |
| Casein hydrolysate | 15 |
| Yeast Extract (dried) | 10 |
| De-foamer Basildon | 0.4 |

The pH was adjusted to 7.0 by adding NaOH/H₂SO₄, and the medium was sterilized by autoclavation (20 minutes at 120°C). The content of a full-grown shake flask was used to inoculate a fermentation vessel containing 6L medium of the following composition:

| | g/L |
|---|---|
| Soybean flower | 25 |
| Soybean oil | 8 |
| Com Steep (dried) | 1 |
| KH₂PO₄ | 0.45 |
| Trace element solution | 17 |
| De-foamer Basildon | 0.4 |

The composition of the trace element solution was as follows:

| | g/L |
|---|---|
| Citric acid. 1H₂O | 175 |
| FeSO₄.7H₂O | 5.5 |
| MgSO₄.7H₂O | 100 |
| H₃BO₃ | 0.06 |
| CuSO₄.5H₂O | 0.13 |
| ZnSO₄.7H₂O | 1.3 |
| CoSO₄.7H₂O | 0.14 |

The temperature and pH of the medium were controlled at 25°C and 7.0 respectively. Dissolved oxygen concentration was kept above 30% of air saturation, by increasing airflow and/or stirrer speed when necessary. After preliminary growth in batch culture for approximately 24 hours the culture entered the second phase of fermentation. During the second phase, the growth and product formation were continued by feeding pure soybean oil. A second feeding line was installed to feed ammonia. The average feeding rate of the soybean oil was 3 g/h. Ammonia was supplied in proportion to the soybean oil feeding rate. A series of fermentations were carried out, in which different ammonia feeding rates were applied while keeping the soybean oil feeding rate constant. For this strain, the carbon source and the nitrogen source were totally consumed when the ratio of NH3 to oil was in the range of 30-40 mg NH3/g oil. This condition of C-N double limitation resulted in cultures with the lowest specific viscosities. Nitrogen excess (NH3/oil ratio >40 mg/g) resulted in a considerable increased viscosity of the culture. Carbon excess (NH3/oil ratio <30 mg/g) had a similar effect. In addition, the accumulation of oil had a negative effect on the culture viability. The range of ratios of nitrogen containing nutrients versus carbon containing nutrients is dependent on the strain and the nature of the nitrogen and carbon sources. For every new process, the optimal range can therefore be determined by the present procedure.

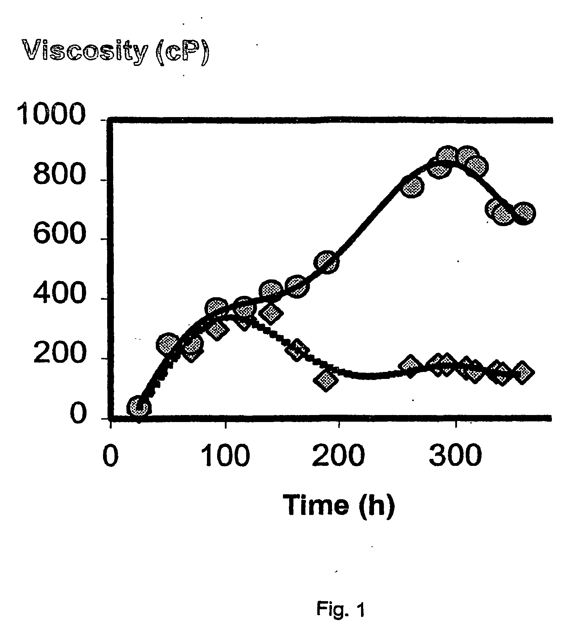

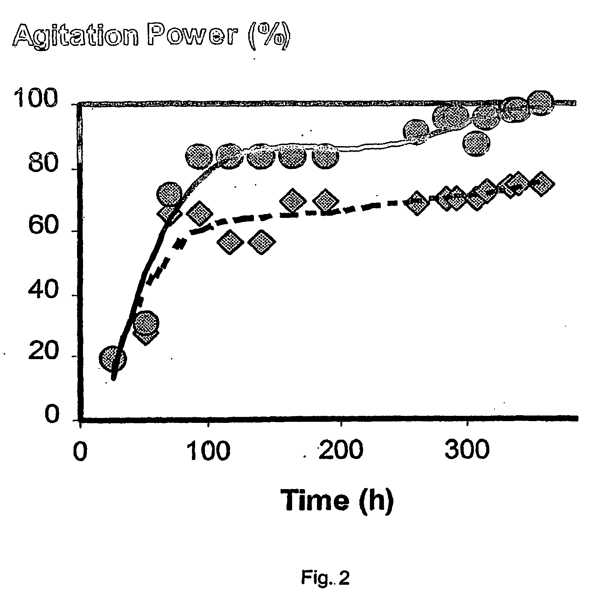

Two experiments were carried out according to the process described above. One experiment was aimed to reach a condition of nitrogen excess (i.e. the culture is then purely limited by the soybean oil feeding rate). In another experiment the rate of ammonia feeding relative to soybean oil feeding was reduced, in order to arrive at a condition where the concentration of both nutrients (soybean oil and ammonia) in the fermenter vessel is very low. For the test organism *(Streptomyces natalensis)* in the chosen conditions, the ratio of ammonia feeding rate relative to the oil-feeding rate should be around 35 mg NH₃ per g oil. The ammonia surplus experiment was carried out at a ratio of 45 mg NH₃ per g oil.
The effect of the carbon-nitrogen double limitation is clearly demonstrated in Figure 1. Under nitrogen excess conditions the viscosity reaches the usual high values. Under conditions of simultaneous carbon and nitrogen limitation, the viscosity drops to a much lower value, causing better aeration conditions. For a good production it is preferred that the dissolved oxygen concentration is maintained at a level of above 30% of air saturation. Figure 2 illustrates that for maintaining this dissolved oxygen concentration much less agitation power (energy) is needed when the culture is under a condition of nitrogen-carbon double limitation.

### Example 2

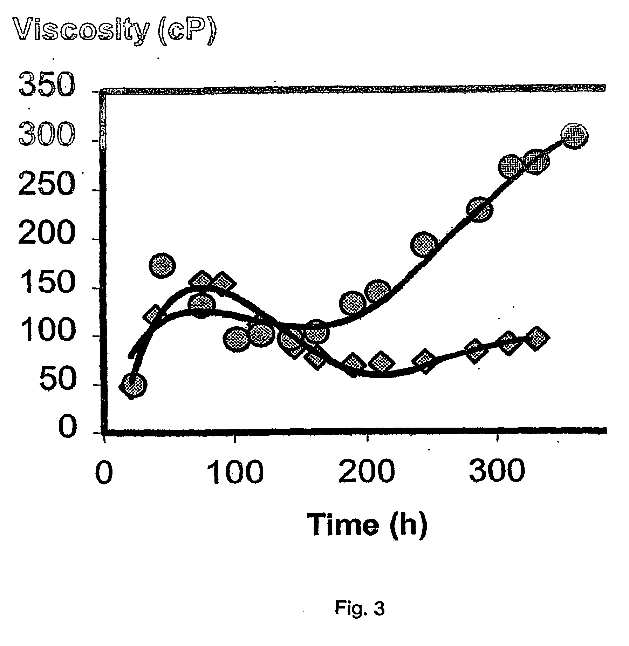

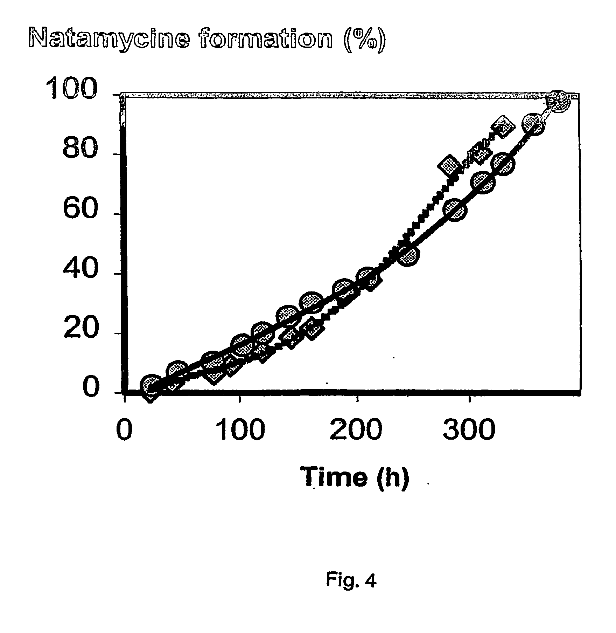

Another fermentation experiment was carried out using the same procedure as described in Example 1 using a strain of *Streptomyces natalensis.* This strain is a producer of the anti-fungal compound natamycin. In this experiment two fermentations were run. One experiment was under carbon limitation and nitrogen excess (NH₃ level was kept at 150-200 mg/L during the oil feeding phase). The second experiment was run under nitrogen-carbon double limitation during the oil feeding phase, employing a NH₃/oil ratio of 32 mg/g. Some results are shown in Figure 3 and 4. It is obvious that a very significant difference in viscosity was observed between the two modes of fermentation. A low viscosity is very beneficial for efficient process operation. However, a low viscosity coupled with a poor product formation potency would be negative. In this experiment, the product formation was not affected at all by the conditions leading to low viscosity (Figure 3). The rate of product formation in the nitrogen-carbon double limitation experiment is faster in the second part of the fermentation despite a slightly slower start.

### Example 3

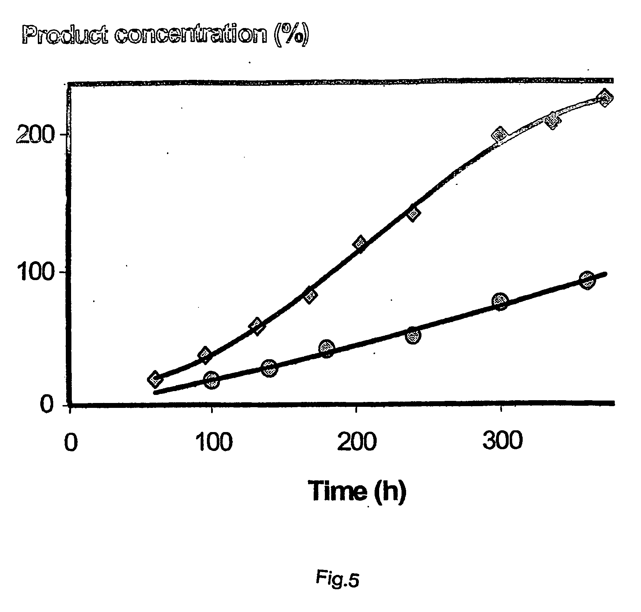

The information obtained in the experiments described in Examples 1 and 2 was used to improve the actual production process of natamycin on an industrial scale (100m³ scale). The reduced viscosity allows intensification of the process by faster feeding of the main nutrient soybean oil. The feeding rate of NH3 was proportional to the feeding of oil, as described in the Examples 1 and 2, resulting in carbon-nitrogen double limitation during the feeding phase (which started at about 24 hours after inoculation of the fermentation vessel). The process conditions and medium composition were similar to the small scale experiments described in Examples 1 and 2. Starting with a small increase, the oil feeding rate was increased step-wise from run to run, until a process intensity was reached which could just be maintained on minimal dissolved oxygen tension. Figure 5 illustrates, the improvement in product output resulting from the higher oil feeding rate was quite substantial.

## Claims

1. A fermentation process for the production of natamycin comprising cultivating *Streptomyces natalensis* or *Streptomyces gilvosporeus* in a liquid fermentation medium comprising a nitrogen and a carbon containing nutrient, wherein a feed comprising carbon containing nutrients and nitrogen containing nutrients is supplied to the medium and wherein the nutrients in the feed are such that the concentration of the nitrogen containing nutrient in the medium is less than 0.5 g/l (expressed as gram of nitrogen per litre) and the concentration of the carbon containing nutrient in the medium is less than 5 g/l (expressed as gram of carbon per litre).

2. A fermentation process according to claim 1, wherein the feed is supplied to the medium via more than one subfeed and wherein each subfeed comprises nitrogen containing nutrients, carbon containing nutrients or a combination of nitrogen and carbon containing nutrients.

3. A fermentation process according to claim 1 or 2, wherein the amount of oxygen in the medium is between 20 and 70% of air saturation.

4. A fermentation process according to claim 3, wherein the amount of oxygen in the medium is between 30 and 60% of air saturation.

5. A fermentation process according to any one of claims 1 to 4, wherein the carbon containing nutrient is for more than 50% soybean oil (calculated as gram of carbon) and the nitrogen containing nutrient is for more than 50% ammonia (calculated as gram of nitrogen).

## Patentansprüche

1. Fermentationsprozess für die Produktion von Natamycin, umfassend die Kultivierung von *Streptomyces natalensis* oder *Streptomyces gilvosporeus* in einem flüssigen Fermentationsmedium, das einen stickstoff- und einen kohlenstoffhaltigen Nährstoff umfasst, wobei das Medium mit einem Zulauf versorgt wird, der kohlenstoffhaltige Nährstoffe und stickstoffhaltige Nährstoffe umfasst und wobei die Nährstoffe in dem Zulauf derart sind, dass die Konzentration des stickstoffhaltigen Nährstoffs in dem Medium weniger als 0,5 g/l (ausgedrückt als Gramm Stickstoff pro Liter) und die Konzentration des kohlenstoffhaltigen Nährstoffs in dem Medium weniger als 5 g/l (ausgedrückt als Gramm Kohlenstoff pro Liter) beträgt.

2. Fermentationsprozess nach Anspruch 1, wobei das Medium mit dem Zulauf über mehr als einen Teilzulauf versorgt wird und wobei jeder Teilzulauf stickstoffhaltige Nährstoffe, kohlenstoffhaltige Nährstoffe oder eine Kombination von stickstoff- und kohlenstoffhaltigen Nährstoffen umfasst.

3. Fermentationsprozess nach Anspruch 1 oder 2, wobei die Sauerstoffmenge in dem Medium zwischen 20 und 70% Luftsättigung beträgt.

4. Fermentationsprozess nach Anspruch 3, wobei die Sauerstoffmenge in dem Medium zwischen 30 und 60% Luftsättigung beträgt.

5. Fermentationsprozess nach einem der Ansprüche 1 bis 4, wobei es sich bei dem kohlenstoffhaltigen Nährstoff um mehr als 50% Sojabohnenöl (berechnet als Gramm Kohlenstoff) und bei dem stickstoffhaltigen Nährstoff um mehr als 50% Ammoniak (berechnet als Gramm Stickstoff) handelt.

## Revendications

1. Procédé de fermentation pour la production de natamycine comprenant la culture de *Streptomyces natalensis* ou *Streptomyces gilvosporeus* dans un milieu de fermentation liquide comprenant un nutriment contenant de l'azote et du carbone, **caractérisé en ce qu'**un substrat comprenant des nutriments contenant du carbone et des nutriments contenant de l'azote est apporté dans le milieu et **en ce que** les nutriments dans le substrat sont tels que la concentration du nutriment contenant de l'azote dans le milieu est inférieure à 0,5 g/l (exprimée en gramme d'azote par litre) et la concentration du nutriment contenant du carbone dans le milieu est inférieure à 5 g/l (exprimée en gramme de carbone par litre).

2. Procédé de fermentation selon la revendication 1, **caractérisé en ce que** le substrat est apporté dans le milieu par l'intermédiaire de plus d'un sous-substrat et **en ce que** chaque sous-substrat comprend des nutriments contenant de l'azote, des nutriments contenant du carbone ou une combinaison de nutriments contenant de l'azote et du carbone.

3. Procédé de fermentation selon la revendication 1 ou 2, **caractérisé en ce que** la quantité d'oxygène dans le milieu est comprise entre 20 et 70 % de saturation d'air.

4. Procédé de fermentation selon la revendication 3, **caractérisé en ce que** la quantité d'oxygène dans le milieu est comprise entre 30 et 60 % de saturation d'air.

5. Procédé de fermentation selon l'une quelconque des revendications 1 à 4, **caractérisé en ce que** le nutriment contenant du carbone est à plus de 50 % de l'huile de soja (calculé en gramme de carbone) et le nutriment contenant de l'azote est à plus de 50 % de l'ammoniac (calculé en gramme d'azote).
